(19) Europäisches Patentamt / European Patent Office / Office européen des brevets

(11) **EP 4 372 364 A1**

(12) **EUROPÄISCHE PATENTANMELDUNG**

(43) Veröffentlichungstag:
    **22.05.2024   Patentblatt 2024/21**

(21) Anmeldenummer: **23210407.5**

(22) Anmeldetag: **16.11.2023**

(51) Internationale Patentklassifikation (IPC):
    **G01N 21/31** (2006.01)    **G01N 21/84** (2006.01)
    **G01N 21/01** (2006.01)    **G01N 21/33** (2006.01)
    **G01N 21/47** (2006.01)

(52) Gemeinsame Patentklassifikation (CPC):
    **G01N 21/8422; G01N 21/31;** G01N 21/33;
    G01N 2021/0118; G01N 2021/3155;
    G01N 2021/3181; G01N 2021/4709;
    G01N 2021/4742; G01N 2021/4745;
    G01N 2021/8433; G01N 2201/0221;
    G01N 2201/024; G01N 2201/062

(84) Benannte Vertragsstaaten:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Benannte Erstreckungsstaaten:
    **BA**
    Benannte Validierungsstaaten:
    **KH MA MD TN**

(30) Priorität: **21.11.2022   DE 102022130758
    27.03.2023   DE 102023107705**

(71) Anmelder: **Courage + Khazaka electronic GmbH
    50829 Köln (DE)**

(72) Erfinder:
    • **KHAZAKA, Georg
      50859 Köln (DE)**
    • **REBLE, Carina
      50829 Köln (DE)**
    • **KHAZAKA-OHMANN, Diana
      50859 Köln (DE)**
    • **WIORA, Georg
      50733 Köln (DE)**

(74) Vertreter: **Dantz, Dirk
    Dantz IP & Law
    Kurfürstendamm 43
    10719 Berlin (DE)**

(54)   **VORRICHTUNG UND VERFAHREN ZUR BESTIMMUNG EINES SCHUTZFAKTORS**

(57)   Die Erfindung betrifft ein Schutzfaktor-Evaluierungssystem zur Bestimmung eines Schutzfaktors eines Hautschutzmittels mit einer Strahlungsquelle mit genau einer LED, einer Detektoreinheit mit genau einer Photodiode einer Steuereinheit sowie einer Auswerteeinheit. Weiterhin betrifft die Erfindung ein Verfahren zur Bestimmung eines Sonnenschutzfaktors eines Hautschutzmittels mit den Verfahrensschritten Emittieren von Strahlung aus genau einer LED einer Strahlungsquelle, Detektieren von remittierter Strahlung mit genau einer Photodiode einer Detektoreinheit und Evaluierung des Schutzfaktors in einem Evaluierungswellenlängenbereich, wobei der Schutzfaktor des Schutzmittels aus der Strahlung und eines Transmissionsspektrums evaluiert wird, und wobei die Daten des Transmissionsspektrums zur Bestimmung des Schutzfaktors in-silico- und/oder in vitro Daten sind.

Fig. 1

EP 4 372 364 A1

**Beschreibung**

Die Erfindung betrifft ein Schutzfaktor-Evaluierungssystem zur Bestimmung eines Schutzfaktors eines Hautschutzmittels mit einer Strahlungsquelle mit genau einer LED, einer Detektoreinheit mit genau einer Photodiode einer Steuereinheit sowie einer Auswerteeinheit. Weiterhin betrifft die Erfindung ein Verfahren zur Bestimmung eines Sonnenschutzfaktors eines Hautschutzmittels mit den Verfahrensschritten Emittieren von Strahlung aus genau einer LED einer Strahlungsquelle, Detektieren von remittierter Strahlung mit genau einer Photodiode einer Detektoreinheit und Evaluierung des Schutzfaktors in einem Evaluierungswellenlängenbereich, wobei der Schutzfaktor des Schutzmittels aus der Strahlung und eines Transmissionsspektrums evaluiert wird, und wobei die Daten des Transmissionsspektrums zur Bestimmung des Schutzfaktors in-silicoung/oder in vitro Daten sind.

Stand der Technik

Die bisherigen durch die Behörden der europäischen Union (EU) und der amerikanischen Food and Drug Administration (FDA) zugelassenen Methoden für die Bestimmung des SPF (Sun Protect Factor) sind alle schädigend für den beteiligten Probanden, indem sie ein Erythem, also eine durch Licht hervorgerufene Entzündungsreaktion der Haut hervorrufen (COLI PA -15 European Cosmetic, Toiletry and Perfumery Association: Colipa SPF Test Method 94/289, 1994; ISO-Standards 24442, 24443, 24444). Daher haben sowohl die FDA als auch die EU mehrfach darauf hingewiesen, dass künftige Forschungsaktivitäten auf neue Methoden zur Charakterisierung der Schutzwirkung von Sonnenschutzmitteln gerichtet werden müssen, um Spätfolgen für die Probanden zu vermeiden (European Commission, 20 Standardisation Mandate Assigned To CEN Concerning Methods For Testing Efficacy Of Sunscreen Products, M/389 EN, Brüssels, 12 July 2006).

Diese Aufgabe soll mit dieser Erfindung wahrgenommen werden. Die bestehenden Verfahren sind in verschiedenen Fundstellen definiert:

In Normen und Vorschriften definierte Verfahren:

a. ISO 24444 definiert ein Verfahren zur In-vivo-Bestimmung des SPF. Grundlage des Verfahrens ist die Erzeugung von Erythemen auf der Haut von Probanden durch Strahlung im UVB-Bereich. Daher ist das Verfahren für den Probanden schädigend.

b. ISO 24443 definiert ein In-vitro-Verfahren zur Bestimmung des UVA-Schutzfaktors (UVAPF). Das Sonnenschutzmittel wird auf eine Kunststoffplatte aufgetragen, so dass ein Transmissionsspektrum des Sonnenschutzmittels gemessen werden kann. Aufgrund nicht kontrollierbarer Schwankungen der Prozedur wird das Transmissionsspektrum durch eine Skalierung an das Ergebnis des Erythem-Tests nach ISO 24444 angepasst und ist damit von dessen Durchführung abhängig. Die verwendete Kunststoffplatte ist mit einer aufgerauten Oberfläche ein unrealistisches Hautmodell.

c. ISO 24442 definiert ein In-vivo-Verfahren, in dem der UVA-Schutzfaktor mittels der minimalen UVA-Dosis zur Erzeugung einer irreversiblen Pigmentierung (Sonnenbräune) der Haut bestimmt wird. Auch dieses Verfahren bedingt eine Veränderung der Haut des Probanden.

Patentierte Verfahren:

DE 198 28 497 A1 beschreibt ein Verfahren, in dem wie in der ISO 24444 bei Probanden durch UV-Bestrahlung der Haut Erytheme erzeugt werden. Diese werden im Gegensatz zur ISO 24444 durch Reflexionsspektroskopie detektiert. Das Verfahren ist damit ebenfalls schädigend. Die optische Wirkung (Schutz) des Sonnenschutzmittels wird nicht mit direkten optischen Messungen erfasst, sondern über eine biologische Reaktion des Körpers.

In DE 10 2004 020 644 A1 wird ein Verfahren beschrieben, in dem die Erzeugung von Radikalen durch UV-Belichtung in vivo mittels Elektronenspinresonanz (ESR) quantitativ gemessen wird. Auch hier wird die optische Wirkung des Sonnenschutzmittels nur indirekt erfasst. Zudem ist die Messung der ESR technisch aufwändig und erfordert relativ große, stationäre Geräte (Tischgeräte). Auch sind sie empfindlich auf Störungen durch Hochfrequenz-Einstrahlungen oder schnelle temporäre Magnetfeldänderungen, wie z.B. von elektrischen Schaltvorgängen.

Um den Label-SPF von topisch applizierten Sonnenschutzmitteln in vivo zu ermitteln, werden weltweit Testmethoden wie die ISO 24444, die FDA-Guideline oder der Australische Standard verwendet. Grundlage all dieser Methoden ist das Herbeiführen einer erythemalen Hautreaktion durch das Bestrahlen der Haut mit UV-Licht. Dies ist erforderlich, um die minimale erythemale Dosis der unbehandelten (MEDu) und produktbehandelten Haut (MEDp) zu bestimmen. Zuverlässige in vitro Methoden, bei denen die menschliche Haut durch synthetische Substratträger ersetzt wird, sind für die SPF-Bestimmung nicht verfügbar.

Bekannt sind monochromatische Geräte, die herkömmliche Xenon-Lampen verwenden und daher teuer in Anschaffung und Betrieb sind. Durch verbaute Monochromatoren werden unterschiedliche Wellenlängen nacheinander gemessen, was bei Bewegungen der Probanden unvorteilhaft ist. Polychromatische Geräte von verwenden ebenfalls Xenon-Lampen. Der in vivo Messwert wird mittels Filter derart gewichtet, dass es mit dem in vivo UVA-PF übereinstimmt. Multi-LED-Geräte für Testinstitute stellen eine weitere Variante da, welche jedoch durch die spektroskopische Detektion deutlich größer und teurer ist.

Es ist daher Aufgabe der Erfindung, ein Verfahren zur Bestimmung eines Schutzfaktors eines Hautschutzmittels bereitzustellen, mit dem die Belastung infolge der Einstrahlung auf die menschliche Haut verringert wird, das qualitativ hochwertige Analyseergebnisse bereitstellt und gleichzeitig schnell und einfach durchführbar ist. Es ist ebenfalls Aufgabe der Erfindung, ein Schutzfaktor-Evaluierungssystem zur Untersuchung des Sonnenschutzfaktors von Sonnenschutzmitteln bereitzustellen, das qualitativ hochwertige Analyseergebnisse bereitstellt, die Belastung infolge der Einstrahlung auf die menschliche Haut verringert und kostengünstig in Herstellung und Betrieb ist.

Die Aufgabe wird mittels des Schutzfaktor-Evaluierungssystems zur Bestimmung eines Schutzfaktors eines Hautschutzmittels gemäß Anspruch 1 gelöst. Weitere vorteilhafte Ausführungen der Erfindung sind in den Unteransprüchen dargelegt.

Das erfindungsgemäße Schutzfaktor-Evaluierungssystem zur Bestimmung eines Schutzfaktors eines Hautschutzmittels weist eine Strahlungsquelle mit genau einer LED auf. Erfindungsgemäß erfolgt die Messung und Erfassung der elektromagnetischen Strahlung nicht mit einem Summenspektrum erzeugt von unterschiedlichen Strahlquellen, sondern mit nur genau einer Strahlquelle, wobei die Strahlquelle eine LED ist. Dadurch ist das erfindungsgemäße Schutzfaktor-Evaluierungssystem deutlich kompakter und kostengünstiger als bekannte Systeme.

Außerdem weist das Schutzfaktor-Evaluierungssystem eine Detektoreinheit mit genau einer Photodiode auf. Die Detektoreinheit umfasst z.B. einen Monochromator, Filter, Photomultiplier, Spektrometer und/oder eine Photodiode. Alle diese genannten Vorrichtungen sind geeignet, elektromagnetische Strahlung zu detektieren und ihre Intensität abhängig von der Wellenlänge zu erfassen. Die Detektion einer in vivo-Messung erfolgt mit einer Photodiode. Dadurch ist das erfindungsgemäße Schutzfaktor-Evaluierungssystem kompakter und kostengünstiger als bekannte Systeme.

Das Schutzfaktor-Evaluierungssystem weist weiterhin eine Steuereinheit sowie eine Auswerteeinheit auf. Das Detektionsfenster und die Auflösung des Spektrometers werden mittels der Steuereinheit festgelegt, die detektierten Signale mittels der Steuereinheit gespeichert, aufbereitet (z.B. verstärkt) und dargestellt. Die Auswerteeinheit ist z.B. ein Computer und weist ein geeignetes Computerprogramm zur Bestimmung eines Schutzfaktors auf.

In einer vorteilhaften Weiterbildung der Erfindung sind die Strahlungsquelle und die Detektoreinheit in einer ersten Baueinheit zusammengefasst. Weitere Komponenten, z.B. eine Auswerteeinheit, sind nicht in der ersten Baueinheit angeordnet. Die Abmessungen und Masse der ersten Baueinheit sind durch diese vorteilhafte Bauweise begrenzt, kompakt und derart gering, dass ein Nutzer mit beiden Händen, vorzugsweise mit einer Hand die erste Baueinheit ohne weitere Hilfsmittel tragen, halten und bedienen kann.

In einer weiteren Ausführung der Erfindung weist die erste Baueinheit eine erste Kommunikationseinheit auf. Mittels der ersten Kommunikationseinheit sind die Daten der von der Photodiode aufgenommenen remittierten und/oder transmittierten elektromagnetischen Strahlung an die Auswerteeinheit versendbar und Daten von der externen Auswerteeinheit empfangbar. Mittels der Auswerteeinheit ist der Schutzfaktor eines Schutzmittels bestimmbar.

In einer weiteren Gestaltung der Erfindung sind die erste Baueinheit und die Steuereinheit oder die Auswerteeinheit in unterschiedlichen Einhausungen angeordnet. In einer Weiterbildung der Erfindung weisen die unterschiedlichen Einhausungen keine strukturelle Verbindung miteinander auf. Die Einhausungen schützen die jeweiligen Komponenten vor Witterungseinflüssen. Gleichzeitig können erste Baueinheit, Steuereinheit und Auswerteeinheit in jeweils voneinander unabhängig angeordneten Einhausungen angeordnet werden, wodurch die einzelnen Komponenten resilient gegenüber Beschädigungen und/oder Softwareausfällen sind.

In einer weiteren Ausbildung der Erfindung weist die erste Baueinheit die Steuereinheit auf. Mittels der ebenfalls in der ersten Baueinheit angeordneten ersten Steuereinheit ist die erste Baueinheit steuerbar, außerdem ist die von der Photodiode erfasste Strahlung mittels der ersten Steuereinheit verarbeitbar. Das Detektionsfenster und die Auflösung der Detektionseinheit wird mittels der ersten Steuereinheit festgelegt, die detektierten Signale gespeichert und aufbereitet (z.B. verstärkt).

In einer weiteren Ausführung der Erfindung ist die Auswerteeinheit in einer zweiten Baueinheit angeordnet. Die Auswerteeinheit in der zweiten Baueinheit ist vorteilhafterweise in einem räumlichen Abstand von der ersten Baueinheit angeordnet. Mittels der ersten Kommunikationseinheit, die mit der ersten Steuereinheit verbunden ist, sind die Daten der von der Photodiode aufgenommenen remittierten und/oder transmittierten elektromagnetischen Strahlung an die Auswerteeinheit versendbar und Daten von der externen Auswerteeinheit empfangbar. Mittels der Auswerteeinheit ist der Schutzfaktor eines Schutzmittels bestimmbar.

In einer weiteren Gestaltung der Erfindung weist die zweite Baueinheit eine zweite Kommunikationseinheit auf. In einer weiteren Ausgestaltung der Erfindung sind die erste Kommunikationseinheit und die zweite Kommunikationseinheit geeignet, miteinander zu kommunizieren. In einer weiteren Ausführung der Erfindung sind die erste und die zweite Kommunikationseinheit dafür geeignet, kabellos miteinander zu kommunizieren. Die kabellose Verbindung zwischen erster und zweiter Kommunikationseinheit ermöglicht eine flexible Positionierung von erster Baueinheit und zweiter Baueinheit unabhängig voneinander. In einer Weiterbildung der Erfindung sind die erste und/oder die zweite Kommunikationseinheit dafür geeignet, über ein öffentliches Netzwerk miteinander zu kommunizieren. In einer weiteren Gestaltung der Erfindung ist das öffentliche Netzwerk ein Mobilfunk-, ein WLAN-, NFC- Netz und/oder eine Bluetooth-Verbindung. Erste und zweite Kommunikationseinheit nutzen daher bereits vorhandene Netzwerke mit etablierten Schnittstellen.

In einer weiteren Ausbildung der Erfindung ist die LED geeignet, Licht mit einer Wellenlänge im UVA-Bereich (Bestrahlungswellenlänge 320nm bis 400nm) zu emittieren. Das Aussenden der Strahlung erfolgt bevorzugt in vivo auf die menschliche Haut gemäß ISO 24442. ISO 24442 definiert ein In-vivo-Verfahren, in dem der UVA-Schutzfaktor mittels der minimalen UVA-Dosis zur Erzeugung einer irreversiblen Pigmentierung (Sonnenbräune) der Haut bestimmt wird.

In einer vorteilhaften Weiterbildung der Erfindung ist die erste Baueinheit ein Handgerät. Ein Handgerät im Sinne der Erfindung ist ein Gerät, das einfach und komfortabel auch über einen längeren Zeitraum zu halten und zu bedienen ist. Das Handgerät weist derartige Abmessungen und eine derartige Masse auf, dass ein Nutzer das Handgerät mit beiden Händen tragen, halten und bedienen kann. Vorzugsweise weist das Handgerät derartige Abmessungen und eine derartige Masse auf, dass ein Nutzer das Handgerät mit einer Hand tragen, halten und bedienen kann.

Die erste Baueinheit weist lediglich die Komponenten zur Erzeugung und Detektion von Strahlung und deren Steuerung auf. Weitere Komponenten, z.B. eine Auswerteeinheit, sind nicht im Handgerät angeordnet. Die Abmessungen und Masse der ersten Baueinheit sind durch diese vorteilhafte Bauweise begrenzt, kompakt und derart gering, dass ein Nutzer mit beiden Händen, vorzugsweise mit einer Hand die erste Baueinheit ohne weitere Hilfsmittel tragen, halten und bedienen kann. Die Masse der ersten Baueinheit beträgt maximal 1000g, bevorzugt maximal 500g und besonders bevorzugt maximal 200g. Die erste Baueinheit ist daher geeignet, dass ein Nutzer die erste Baueinheit komfortabel transportieren und mehrfache Bestimmungen eines Schutzfaktors eines Hautschutzmittels auch über einen längeren Zeitraum durchführen kann.

In einem weiteren Aspekt der Erfindung ist die zweite Baueinheit ein Mobilgerät und bevorzugt ein Smartphone. Das Mobilgerät kann auch ein Notebook-Computer, Tablet o.ä. sein und ermöglicht eine flexible Positionierung von erster Baueinheit und Auswerteeinheit unabhängig voneinander. Das Mobilgerät bzw. Smartphone weist eine geeignete Applikation zur Bestimmung des Schutzfaktors eines Schutzmittels auf.

Die Aufgabe wird außerdem mittels des erfindungsgemäßen Verfahrens zur Bestimmung eines Schutzfaktors eines Hautschutzmittels gelöst. Weitere vorteilhafte Ausführungen der Erfindung sind ebenfalls in den Unteransprüchen dargelegt.

Das erfindungsgemäße Verfahren zur Bestimmung eines Schutzfaktors eines Hautschutzmittels weist drei Verfahrensschritte auf: Im ersten Verfahrensschritt erfolgt ein Emittieren von Strahlung aus genau einer LED einer Strahlungsquelle. Die LED erzeugt elektromagnetische Strahlung mit einer Bestrahlungswellenlänge aus dem blauen Spektralbereich (von etwa 400nm bis 500nm Wellenlänge) und/oder dem UV-Bereich (280nm bis 400nm).

Die Wellenlängenbereich sind wie folgt definiert:

- 280-320 nm (UVB) mit < 0,1 % der gesamten UV-Intensität,
- 320 nm bis 340 nm (UVA II) 8% bis 20 % der gesamten UVA-Intensität
- 340 nm bis 400 nm (UVA I) 80 % bis 92 % der gesamten UVA-Intensität
- 400 nm bis 500 nm, blaues Licht

Im zweiten Verfahrensschritt erfolgt ein Detektieren von remittierter Strahlung von genau einer Photodiode einer Detektoreinheit. Das Verhältnis der Intensitäten der remittierten Strahlung zur in den Messkörper eingekoppelten Strahlung ist ein Maß der Schutzfähigkeit des Schutzmittels. Die Detektionswellenlänge umfasst wie die Bestrahlungswellenlänge bevorzugt einen Wellenlängenbereich, wobei der Wellenlängenbereich der Detektionswellenlänge bevorzugt innerhalb des Bereichs der Bestrahlungswellenlänge liegt oder den gesamten Bereich der Bestrahlungswellenlänge umfasst.

Im dritten Verfahrensschritt erfolgt eine Evaluierung des Schutzfaktors in einem Evaluierungswellenlängenbereich, wobei der Schutzfaktor des Schutzmittels aus der Strahlung und eines Transmissionsspektrums evaluiert wird. Zusätzlich sind die Daten des Transmissionsspektrums zur Bestimmung des Schutzfaktors in-silico- und/oder in vitro Daten. Aufgrund der hohen Absorptionseigenschaften gibt die menschliche Haut nicht genügend UVB-Strahlung ab, um das Absorptionsspektrum des aufgetragenen Produkts im UVB-Bereich zu messen. Es ist daher notwendig, das Absorptionsspektrums des Testmaterials im UVB-Teil des Spektrums (280-320 nm) separat mit einer anderen Technik zu erfassen. Ein Transmissionsspektrum wird auf Grundlage der der UV-Durchlässigkeit von Schutzfilmen in vitro ermittelt. Die Substrate, auf die die Schutzfilme aufgetragen werden, bilden nur annähernd die inhomogene Oberflächenstruktur der menschlichen Haut ab, wie z.B. Polymethylmethacrylat (PMMA)-Platten mit rauer Oberfläche nach ISO 24443. Die Daten des Transmissionsspektrums beinhalten Intensität über Wellenlänge in vorzugsweise digitalisiertem Format. In einer weiteren Ausgestaltung der Erfindung sind die Daten des Transmissionsspektrums in-silico-Daten. Solche Daten des Transmissionsspektrums sind also weder in vivo an einem Probanden ermittelt noch in vitro nach ISO 24443, sondern rechnerisch abgeschätzt bzw. ermittelt. Wenn die Eigenschaften der Filtersubstanzen eines Schutzmittels bekannt sind, kann die Transmission berechnet und simuliert werden. Anhand der simulierten Transmission können der Lichtschutzfaktor und alle Größen, die den Schutzfaktor charakterisieren, berechnet werden.

In einer Weiterbildung der Erfindung werden Daten der detektierten remittierten Strahlung zu einer Auswerteeinheit übertragen. Die Auswerteeinheit ist z.B. ein Computer und weist ein geeignetes Computerprogramm auf.

In einer weiteren Ausführung der Erfindung ist die Auswerteeinheit in ein anderen strukturellen Baueinheit angeordnet als die Strahlungsquelle und/oder die Detektoreinheit. In einer weiteren Ausgestaltung der Erfindung ist die Auswerteeinheit ein Computer oder ein Mobilfunkgerät. Der Computer bzw. das Mobilfunkgerät ist vorzugsweise entfernt von der Strahlungsquelle und/oder der Detektoreinheit angeordnet und weist ein geeignetes Computerprogramm zur Bestimmung eines Schutzfaktors auf. Die Auswerteeinheit selbst kann daher ein handelsüblicher Computer bzw. ein handelsübliches Mobilfunkgerät sein, z.B. ein Smartphone.

In einer weiteren Gestaltung der Erfindung umfasst die emittierte Strahlung einen Bestrahlungswellenlängenbereich zwischen 280nm und 2000nm, bevorzugt 280nm bis 800 nm und besonders bevorzugt den Bereich von 280nm bis 500nm. Die LED ist im Rahmen dieser Schrift eine technische Einrichtung zur Erzeugung von elektromagnetischer Strahlung. Eine LED ist daher nicht ein optisches Element zur Leitung, Umlenkung oder Veränderung der Intensität und/oder Wellenlänge der elektromagnetischen Strahlung. Eine Strahlquelle ist also z.B. kein Lichtleiter, Gitter, Prisma, Filter. Die LED erzeugt elektromagnetische Strahlung mit einer Bestrahlungswellenlänge zwischen dem blauen Spektralbereich (von etwa 400nm bis 500nm Wellenlänge) und dem UV-Bereich (280nm bis 400nm).

In einer weiteren Ausbildung der Erfindung ist der Evaluierungswellenlängenbereich zum Bestrahlungswellenlängenbereich unterschiedlich. In einer weiteren Ausführung der Erfindung umfasst die Evaluierungswellenlänge den Wellenlängenbereich von 280nm bis 2000nm, bevorzugt den Wellenlängenbereich von 280nm bis 800nm oder den besonders bevorzugt den Wellenlängenbereich von 280nm bis 500nm. In einer Weiterbildung umfasst die Evaluierungswellenlänge einen Wellenlängenbereich von 400nm bis 500nm und bevorzugt von 400nm bis 450nm. Bevorzugt wird der Wellenlängenbereich von blauem Licht, der an den UVA-Bereich (bis 400nm) angrenzt, evaluiert. Zur Bestimmung der Schutzfähigkeit des Schutzmittels im UVB-Wellenlängenbereich (< 320nm) kann dieser Wellenlängenbereich ebenfalls optional evaluiert werden.

In einer weiteren Gestaltung der Erfindung erfolgt die Evaluierung der Schutzfähigkeit des Schutzmittels für Licht in einem Wellenlängenbereich von 400nm bis 500nm in einem von der Evaluierung der Schutzfähigkeit des Schutzmittels für Licht in einem Wellenlängenbereich von 280nm bis 400nm separaten Verfahren. Aufgrund der hohen Absorptionseigenschaften gibt die menschliche Haut nicht genügend UVB-Strahlung ab, um das Absorptionsspektrum des aufgetragenen Produkts im UVB-Bereich zu messen. Es ist daher notwendig, das Absorptionsspektrums des Testmaterials im UVB-Teil des Spektrums (280-320nm) separat mit einer anderen Technik zu erfassen. Dazu wird die in vivo Remission im Wellenlängenbereich von 320nm bis 400nm mit einer Photodiode erfasst, der Wellenlängenbereich von 280nm bis zur LED-Wellenlänge und der Wellenlängenbereich von der LED-Wellenlänge bis 500nm mit einem in silico und/oder in vitro Transmissionsspektrum.

In einer Weiterbildung der Erfindung umfasst der Wellenlängenbereich des Transmissionsspektrums die Evaluierungswellenlänge. Die Evaluierung des Schutzfaktors des Schutzmittels erfolgt aus der in vivo remittierten Strahlung und dem in silico und/oder in vitro Transmissionsspektrum. Aufgrund der hohen Absorptionseigenschaften gibt die menschliche Haut nicht genügend UVB-Strahlung ab, um das Absorptionsspektrum des aufgetragenen Produkts im UVB-Bereich zu messen. Es ist daher notwendig, das Absorptionsspektrums des Testmaterials im UVB-Teil des Spektrums (280-320nm) separat mit einer anderen Technik zu erfassen. Der in dieser Schrift angewandte Ansatz verwendet die in vivo-Auswertung der absoluten UVA-Absorption, wie sie mit einer in vivo Messung gemessen wurde, mit einer Verwendung eines berechneten in silico und/oder in vitro Transmissionsspektrums, um die Schutzfaktor des Schutzmittels für einen Nutzer zu ermitteln. Die Evaluierung und Hybridisierung eines in vivo Remissionswerts mit einem in silico und/oder in vitro Transmissionsspektrum erfolgt, um ein vollständiges UV-Spektrum zu erhalten, so dass die Schutzfaktoren nach den Formeln der gültigen Norm (ISO 24443) berechnet werden. Dazu umfasst der Wellenlängenbereich des Transmissionsspektrums die Evaluierungswellenlänge, die bevorzugt den Bereich von 280nm bis 500nm umfasst.

In einer weiteren Ausbildung der Erfindung ist der Wellenlängenbereich der Bestrahlungswellenlänge oder der Wellenlängenbereich der Detektionswellenlänge kleiner als der Wellenlängenbereich der Evaluierungswellenlänge. Die Wellenlängenbereiche der Bestrahlungswellenlänge und der Detektionswellenlänge sind bevorzugt gleich in einem Wellenlängenbereich von maximal 320nm bis 400nm. Der Wellenlängenbereich der Evaluierungswellenlänge umfasst maximal einen Bereich von 280nm bis 500nm.

In einer Weiterbildung der Erfindung ist der Wellenlängenbereich der Bestrahlungswellenlänge und/oder der Wellenlängenbereich der Detektionswellenlänge kleiner 100nm, bevorzugt kleiner 50nm und besonders bevorzugt kleiner 25nm. Daher ist nur eine Stahlquelle zur Abgabe von elektromagnetischer Strahlung nötig, die einen geringen Wellenlängenbereich der Bestrahlungswellenlänge aufweist. In gleicher Weise ist zur Aufnahme des Remissionsspektrums ein Detektor nötig, der geringen Wellenlängenbereich der Detektionswellenlänge erfassen kann. Strahlquelle und Detektor können daher in Herstellung und Betrieb kostengünstig ausgeführt sein.

In einer weiteren Ausführung der Erfindung umfasst die Bestrahlungswellenlänge und/oder die Detektionswellenlänge nur Licht mit Wellenlängen außerhalb des Wellenlängenbereichs von 400nm bis 450nm. Zur Erfassung einer in vivo Messung wird insbesondere der UVA-Wellenlängenbereich (320nm - 400nm) in den Messkörper eingekoppelt.

In einer weiteren Ausgestaltung der Erfindung wird der Schutzfaktor des Schutzmittels aus der remittierten Strahlung und eines Transmissionsspektrums evaluiert. Die Evaluierung des Schutzfaktors des Schutzmittels erfolgt aus der in vivo remittierten Strahlung und dem in silico und/oder in vitro Transmissionsspektrum. Aufgrund der hohen Absorptionseigenschaften gibt die menschliche Haut nicht genügend UVB-Strahlung ab, um das Absorptionsspektrum des aufgetragenen Produkts im UVB-Bereich zu messen. Es ist daher notwendig, das Absorptionsspektrums des Testmaterials im UVB-Teil des Spektrums (280-320nm) separat mit einer anderen Technik zu erfassen. Der in dieser Schrift angewandte Ansatz verwendet die in vivo-Auswertung der absoluten UVA-Absorption, wie sie mit einer in vivo Messung gemessen wurde, mit einer Verwendung eines berechneten in silico und/oder in vitro Transmissionsspektrums, um die Schutzfaktor des Schutzmittels für einen Nutzer zu ermitteln. Die Evaluierung und Hybridisierung eines in vivo Remissionsspektrums mit einem in silico und/oder in vitro Transmissionsspektrum erfolgt, um ein vollständiges UV-Spektrum zu erhalten, so dass die Schutzfaktoren nach den Formeln der gültigen Norm (ISO 24443) berechnet werden. Dazu umfasst der Wellenlängenbereich des Transmissionsspektrums die Evaluierungswellenlänge, die bevorzugt den Bereich von 280nm bis 500nm umfasst.

In einer vorteilhaften Weiterbildung der Erfindung sind die Strahlungsquelle und/oder die Detektoreinheit in einem Handgerät angeordnet. Ein Handgerät im Sinne der Erfindung ist ein Gerät, das einfach und komfortabel auch über einen längeren Zeitraum zu halten und zu bedienen ist. Das erfindungsgemäße Handgerät weist derartige Abmessungen und eine derartige Masse auf, dass ein Nutzer das Handgerät mit beiden Händen tragen, halten und bedienen kann. Vorzugsweise weist das erfindungsgemäße Handgerät derartige Abmessungen und eine derartige Masse auf, dass ein Nutzer das Handgerät mit einer Hand tragen, halten und bedienen kann.

Das Handgerät ist geeignet, mittels der LED elektromagnetische Strahlung in einen Messkörper einzuleiten, bevorzugt in die menschliche Haut. Die Strahlquelle ist eine LED. Außerdem ist das Handgerät geeignet, mittels der Photodiode die remittierte und/oder transmittierte elektromagnetische Strahlung zu erfassen. Mittels der ersten Steuereinheit ist das Handgerät steuerbar, außerdem ist die von der Photodiode erfasste Strahlung mittels der ersten Steuereinheit verarbeitbar. Das Detektionsfenster und die Auflösung der Detektionseinheit wird mittels der ersten Steuereinheit festgelegt, die detektierten Signale gespeichert und aufbereitet (z.B. verstärkt).

Das erfindungsgemäße Handgerät weist lediglich die Komponenten zur Erzeugung und Detektion von Strahlung und deren Steuerung auf. Weitere Komponenten, z.B. eine Auswerteeinheit, sind nicht im Handgerät angeordnet. Die Abmessungen und Masse des erfindungsgemäßen Handgerätes sind durch diese vorteilhafte Bauweise begrenzt, kompakt und derart gering, dass ein Nutzer mit beiden Händen, vorzugsweise mit einer Hand das Handgerät ohne weitere Hilfsmittel tragen, halten und bedienen kann. Die Masse des Handgerätes beträgt maximal 1000g, bevorzugt maximal 500g und besonders bevorzugt maximal 200g. Das Handgerät ist daher geeignet, dass ein Nutzer das Handgerät komfortabel transportieren und mehrfache Bestimmungen eines Schutzfaktors eines Hautschutzmittels auch über einen längeren Zeitraum durchführen kann.

Ausführungsbeispiele des erfindungsgemäßen Verfahrens zur Bestimmung eines Schutzfaktors und des erfindungsgemäßen Schutzfaktor-Evaluierungssystems sind in den Zeichnungen schematisch vereinfacht dargestellt und werden in der nachfolgenden Beschreibung näher erläutert.

Es zeigen:

Fig. 1:    Schutzfaktor-Evaluierungssystem mit Probenkopf, erster Baueinheit und zweiter Baueinheit
Fig. 2:    Probenkopf mit Strahleinlass und Strahlauslass
Fig. 3:    Schutzfaktor-Evaluierungshandgerät mit einer ersten Steuereinheit und einer Bedieneinheit
Fig. 4:    Schutzfaktor-Evaluierungshandgerät mit einer ersten Steuereinheit, einer Bedieneinheit und einer Ausgabeeinrichtung
Fig. 5:    Schutzfaktor-Evaluierungshandgerät mit einer ersten Steuereinheit
Fig. 6:    Schutzfaktor-Evaluierungshandgerät mit einer ersten Steuereinheit und einer Bedieneinheit
Fig. 7:    Schutzfaktor-Evaluierungssystem, Auswerteeinheit und erste Steuereinheit in Baueinheit innerhalb des Mobilfunkgeräts angeordnet
Fig. 8:    Schutzfaktor-Evaluierungshandgerät mit einer ersten Steuereinheit, einer Bedieneinheit und einer Ausgabeeinrichtung
Fig. 9:    Schutzfaktor-Evaluierungshandgerät mit einer ersten Steuereinheit, einer Bedieneinheit, einer Ausgabeeinrichtung und einer zweiten Steuereinheit
Fig. 10:   Schutzfaktor-Evaluierungssystem, Auswerteeinheit und erste Steuereinheit in Baueinheit innerhalb des Mobilfunkgeräts angeordnet

Fig. 11: Verfahren zur Durchführung einer in vivo Messung zur Evaluierung eines Sonnenschutzfaktors mit Referenz- und Kalibrierungsmessung

Fig. 12: Verfahren zur Durchführung einer in vivo Messung unter Einbeziehung eines in silico und/oder in vitro Spektrums zur Evaluierung eines Sonnenschutzfaktors

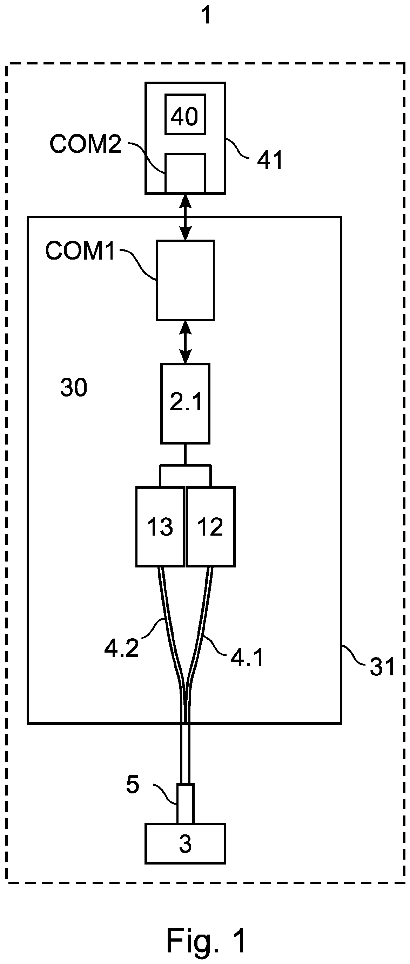

Fig. 1 zeigt schematisch ein Ausführungsbeispiel des erfindungsgemäßen Schutzfaktor-Evaluierungssystems 1 zur Durchführung einer in vivo Messung auf der Haut eines Probanden 3. Das Schutzfaktor-Evaluierungssystem 1 weist die erste Baueinheit 30 auf, die in der ersten Einhausung 31 angeordnet ist. Die erste Baueinheit 30 weist die Strahlquelleneinrichtung 12 sowie die Detektoreinheit 13 auf. Die Strahlenquelleneinrichtung 12 weist eine einzelne LED 12.1 sowie optische Elemente auf, die dafür vorgesehen und/oder dafür geeignet sind, die von der ersten Strahlquelle 12.1 erzeugte erste Strahlung zu konditionieren und/oder umzulenken, z.B. Lichtleiter, Filter, Monochromator, Spiegel und/oder andere optische Elemente.

Die erste Baueinheit 30 weist außerdem die Detektoreinheit 13 auf, die eine einzelne Photodiode 13.1 aufweist. Detektoreinheit 13 und Strahlquelleneinrichtung 12 sind über Datenleitungen mit der ersten Steuereinheit 2.1 verbunden, die ihrerseits über die erste Kommunikationseinheit COM1 mit der Auswerteeinheit 10 verbunden ist. Die Auswerteeinheit 10 ist extern von der ersten Baueinheit 30 in einer zweiten Baueinheit 40 angeordnet, wobei die zweite Baueinheit 40 in einer zweiten Einhausung 41 angeordnet ist. Über die Lichtleiter 4.1, 4.2 ist die erste Baueinheit mit dem Probenkopf 5 verbunden.

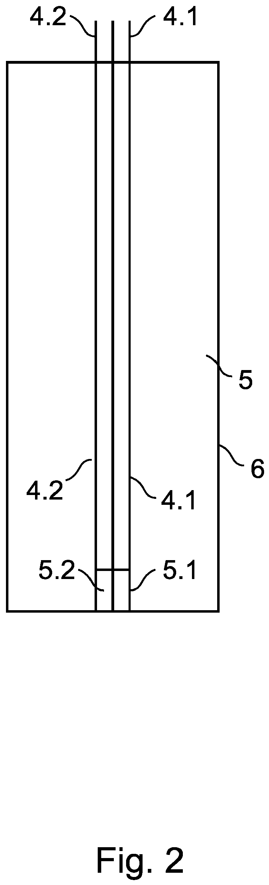

Eine Schnittansicht eines Ausführungsbeispiel eines Probenkopfes 5 zeigt Fig. 2. Der Probenkopf 5 weist den Strahlauslass 5.1 auf, mit dem die von der Messeinrichtung 6 erzeugte elektromagnetische Strahlung über den Lichtleiter 4.1 in die menschliche Haut 3 eingestrahlt wird. Der Probenkopf 5 weist ebenfalls einen Strahleinlass 5.2 auf, mit dem die von dem Messort 3 remittierte Strahlung aufgenommen und über den Lichtleiter 4.2 in die Detektoreinheit 13 geleitet wird.

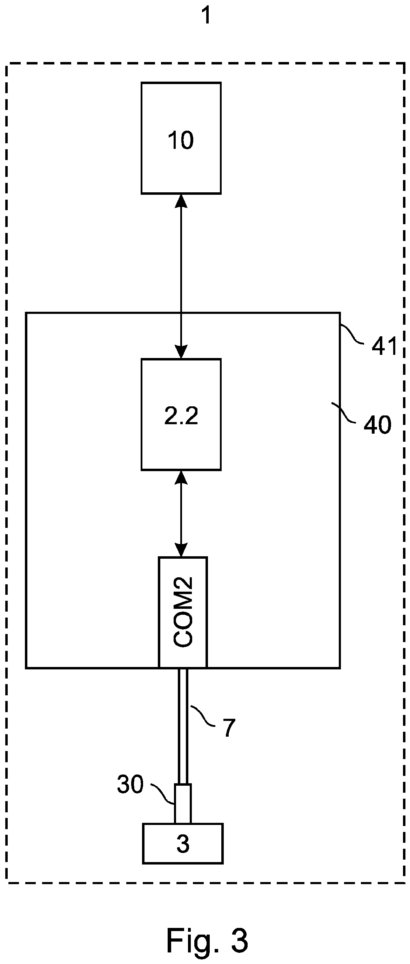

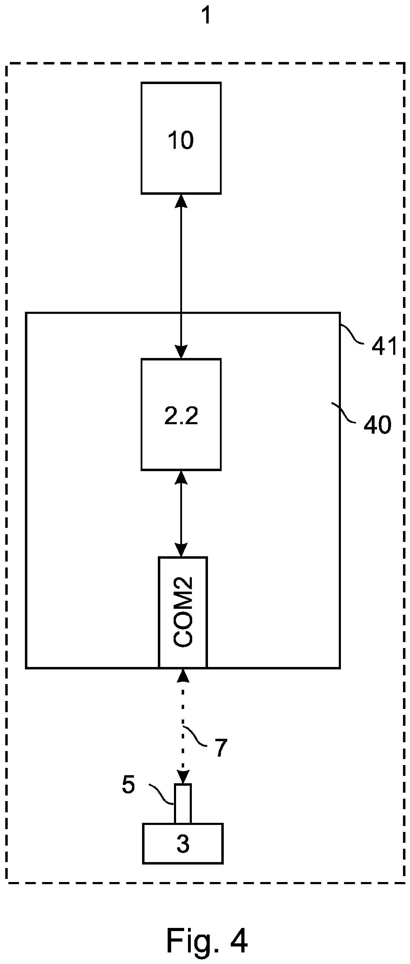

Fig. 3 und Fig. 4 zeigen Ausführungsbeispiele des erfindungsgemäßen Schutzfaktor-Evaluierungssystems 1. Das Schutzfaktor-Evaluierungssystem 1 weist das Handgerät 30 (s. Fig. 1) auf, das zur Bestimmung eines Schutzfaktors eines Hautschutzmittels direkt auf dem Messkörper 3 angeordnet ist. Der Messkörper 3 ist z.B. die Haut eines Probanden. Das Handgerät 30 weist eine erste Kommunikationseinheit COM1 auf, die mit einer zweiten Kommunikationseinheit COM2 eines Mobilfunkgerätes 40 mittels einer Verbindung 7 verbunden ist. Die Verbindung 7 erfolgt durch Kabel oder eine kabellose Bluetooth-Verbindung, eine weitere Möglichkeit der Verbindung ist eine Internetverbindung.

Das Mobilfunkgerät 40, in diesem Ausführungsbeispiel ein Smartphone mit geeigneter App, weist eine zweite Steuereinheit 2.2 auf, die das Verfahren 400 zur Bestimmung eines Schutzfaktors steuert. Die zweite Steuereinheit 2.2 ist mit der zweiten Kommunikationseinheit COM2 verbunden, über die die Daten des Handgerätes 30 empfangen werden. Die Auswerteeinheit 10 ist in diesem Ausführungsbeispiel extern in einem Abstand sowohl von Mobilfunkgerät 40 als auch Handgerät 30 angeordnet. Die Auswerteeinheit 10 ist in diesem Ausführungsbeispiel ein fest angeordneter Desktop-Computer, die Auswerteeinheit 10 kann aber auch ein Mobilgerät sein, z.B. ebenfalls ein Smartphone, Tablet o.ä. und weist eine geeignete App zur Bestimmung eines Schutzfaktors eines Schutzmittels auf.

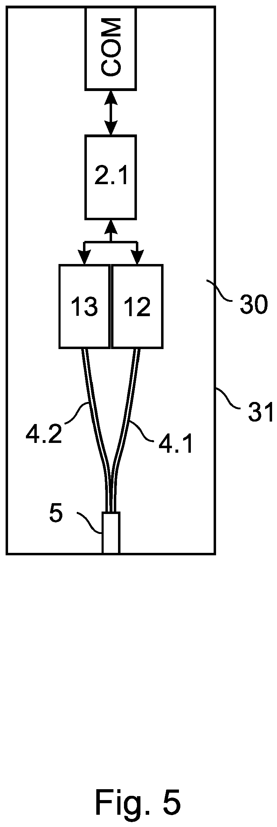

Fig. 5 zeigt ein Ausführungsbeispiel des erfindungsgemäßen Handgeräts 30 zur Durchführung einer in vivo Messung. Das Handgerät 30 weist die Strahlquelleneinrichtung 12 auf. Die Strahlenquelleneinrichtung 12 weist eine LED 12.1 sowie optische Elemente auf, die dafür vorgesehen und/oder dafür geeignet sind, die von der LED 12.1 erzeugte Strahlung zu konditionieren und/oder umzulenken, z.B. Lichtleiter, Filter, Monochromator, Spiegel und/oder andere optische Elemente.

Mittels eines Lichtleiters 4.1 wird das von der Strahlquelleneinrichtung 12 emittierte Licht mit einer Bestrahlungswellenlänge zwischen 280nm und 2000nm über den Probenkopf 5 in den Messkörper 3 eingeleitet. Bevorzugt liegt die Bestrahlungswellenlänge im Bereich von 280nm bis 800nm, besonders bevorzugt im Bereich von 280nm bis 500nm. In diesem und allen weiteren Ausführungsbeispielen liegt die Bestrahlungswellenlänge im Bereich von 280nm bis 500nm (UVA bis blaues Licht). Das von dem Messkörper 3 remittierte Licht gelangt über einen weiteren Lichtleiter 4.2 in die Detektoreinheit 13. Die Detektoreinheit 13 kann einen Monochromator, Filter, Photomultiplier, Spektrometer und/oder eine Photodiode aufweisen. In diesem und allen folgenden Ausführungsbeispielen weist die Detektoreinheit 13 eine Photodiode als Detektor 13.1 auf.

Detektoreinheit 13 und Strahlquelleneinrichtung 12 sind mit einer ersten Steuereinheit 2.1 verbunden, die ihrerseits über eine weitere Datenleitung mit der ersten Kommunikationseinheit COM1 verbunden ist. Die erste Steuereinheit 2.1 ist üblicherweise ein Computerchip mit auf einem Speicher gespeichertem geeigneten Computerprogramm. Die erste Kommunikationseinheit COM1 ist über eine zweite Kommunikationseinheit COM2 (nicht dargestellt) mit einer extern angeordneten Auswerteeinheit 10 kabellos verbunden, wobei erste Kommunikationseinheit COM1 und zweite Kommunikationseinheit COM2 miteinander kommunizieren. Alle genannten Komponenten des Handgeräts 30 sind in einer ersten Einhausung 31 angeordnet, dass die Komponenten vor Verschmutzungen schützt.

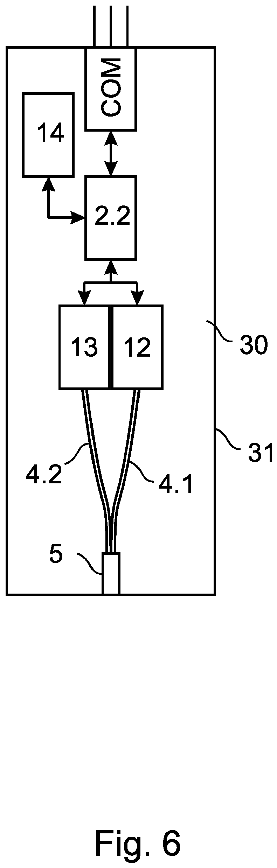

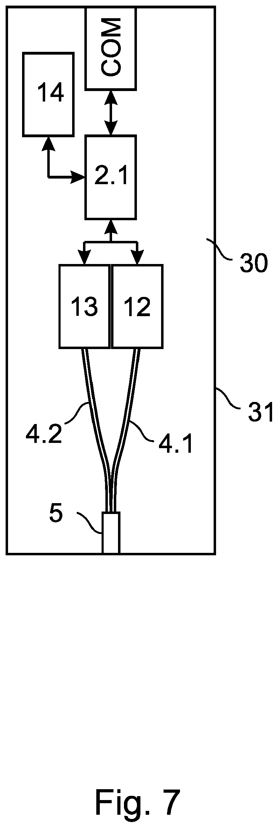

Varianten des Handgeräts 30 zeigen Fig. 6 und Fig. 7. Die hier gezeigten Handgerät 30 entsprechen dem vorherigen Ausführungsbeispiel (s. Fig. 5), lediglich ist die erste Steuereinheit 2.1 (in Fig. 7 2.2) mit einer Bedieneinheit 14 verbunden. Mittels der Bedieneinheit 14 kann ein Nutzer des Handgeräts 30 die erste Steuereinheit 2.1 ansteuern und so das Handgerät 30 bedienen, z.B. die Strahlung erzeugen bzw. abschalten, den Bestrahlungswellenlängenbereich der Strahlquelle 12.1 auswählen und die Intensität der von der Strahlquelle 12.1 erzeugten Strahlung steuern. Erste Kommunikationseinheit COM1 und zweite Kommunikationseinheit COM2 sind mittels einer Kabelverbindung (Fig. 6) oder kabellos (Fig. 7) miteinander verbunden.

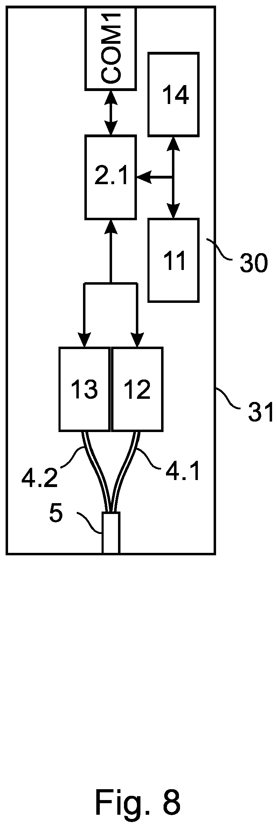

Fig. 8 zeigt ein weiteres Ausführungsbeispiel des Handgeräts 30. Das hier gezeigte Handgerät 30 entspricht dem vorherigen Ausführungsbeispiel (s. Fig. 7), zusätzlich ist die erste Steuereinheit 2.1 mit der Bedieneinheit 14 und einer Ausgabeeinrichtung 11 verbunden. Die Ausgabeeinrichtung 11 ist in diesem Ausführungsbeispiel in dem Handgerät 30 angeordnet. Mittels der Ausgabeeinrichtung 11 ist anzeigbar, ob die LED 12.1 Strahlung aussendet. Alternativ oder zusätzlich sind die Parameter der von der LED 12.1 auszusenden Strahlung und der Photodiode 13.1 anzeigbar. Außerdem ist der bestimmte Schutzfaktor eines Hautschutzmittels anzeigbar.

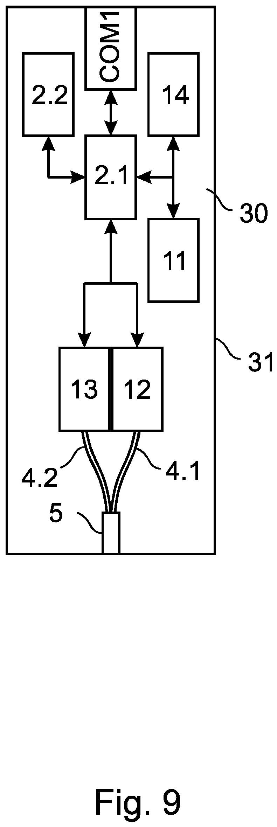

Ein bevorzugtes Ausführungsbeispiel des Handgeräts 30 zeigt Fig. 9. Das hier gezeigte Handgerät 30 entspricht dem vorherigen Ausführungsbeispiel (s. Fig. 4), zusätzlich weist das Handgerät 30 eine zweite Steuereinheit 2.2 auf, die mit der ersten Steuereinheit 2.1 verbunden ist.

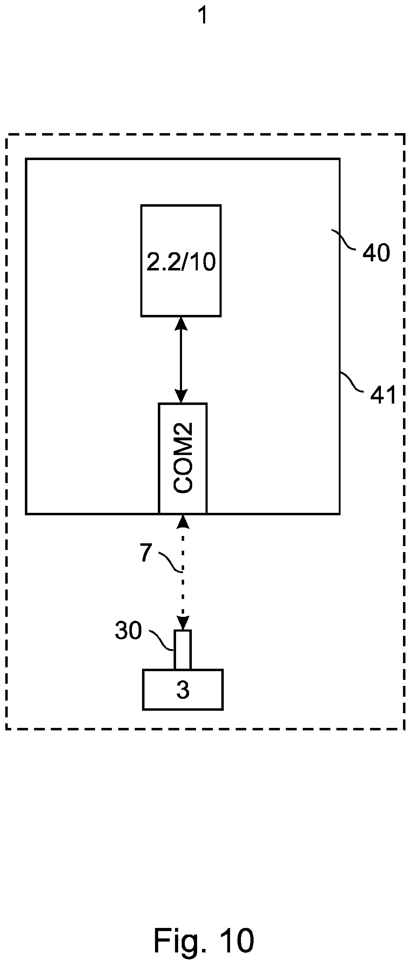

Fig. 10 zeigt ein weiteres Ausführungsbeispiel des erfindungsgemäßen Schutzfaktor-Evaluierungssystems 1. Das Schutzfaktor-Evaluierungssystem 1 weist wie ein vorher gezeigtes Ausführungsbeispiel (s. Fig. 3) das Handgerät 30 auf. Das Handgerät 30 weist eine erste Kommunikationseinheit COM1 auf, die mit einer zweiten Kommunikationseinheit COM2 des Mobilfunkgerätes 6 verbunden ist. Das Mobilfunkgerät 40 weist eine erste Steuereinheit 2.2 auf, die das Verfahren 400 zur Bestimmung eines Schutzfaktors steuert. Die erste Steuereinheit 2.2 ist mit der Kommunikationseinheit COM1 verbunden. Die Auswerteeinheit 10 ist in diesem Ausführungsbeispiel in dem Smartphone 40 in Baueinheit mit der ersten Steuereinheit 2.2 angeordnet.

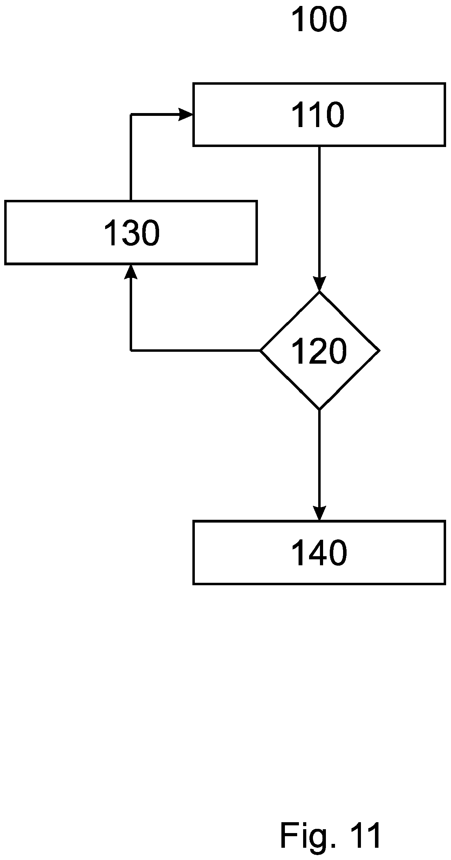

Fig. 11 zeigt ein Ausführungsbeispiel einer Durchführung des Verfahrens 100 einer in vivo Messung zur Erfassung des Remissionsspektrums mittels des erfindungsgemäßen Schutzfaktor-Evaluierungssystems 1, wie in den vorangegangenen Ausführungsbeispielen (s. Fig. 1 bis Fig. 10) gezeigt. Die Durchführung erfolgt in vivo nach ISO 24442 bzw. 24444. Das Verfahren 100 einer Messung erfordert die Aufnahme von jeweils einemRemission der mit Schutzmittel unbehandelten Haut des Probanden 3 und der mit Schutzmittel behandelten Haut des Probanden 3.

Der Probenkopf 5 wird auf die unbehandelte Haut des Probanden 3 aufgebracht, d.h. das zu untersuchende Schutzmittel ist nicht auf der Haut des Probanden 3 appliziert. Dazu wird üblicherweise eine Stelle an der Innenseite des Unterarms oder des Rückens eines Probanden 3 ausgewählt. Danach erfolgt die erste Messung 110, indem die erste Steuereinheit 2.1 die LED 12.1 derart ansteuert, dass das von der LED 12.1 erzeugte Licht durch den Lichtleiter 4.1 auf die Haut des Probanden 3 geleitet wird. Die von der LED 12.1 erzeugte elektromagnetische Strahlung weist einen Bestrahlungswellenlängenbereich mit einer FWHM von bis zu 20nm auf, wobei die erzeugte elektromagnetische Strahlung im Wellenlängenbereich von 330nm bis 350nm liegt. Der Wellenlängenbereich der Detektionswellenlänge, in dem die Photodiode 13.1 die remittierte Strahlung mittels des Lichtleiters 4.2 erfasst, weist ebenfalls einen Bereich von 20nm im Bereich von 330nm bis 350nm auf.

Das von der LED 12.1 erzeugte Licht wird ungefiltert auf den Messkörper 3 eingestrahlt, um ein hohes S / R-Verhältnis zu gewährleisten. Insbesondere ist das von der LED 12.1 erzeugte Licht polychromatisch mit einem Intensitätsmaximum bei einer Wellenlänge im UVA-Bereich von 340nm. Alternativ kann eine LED 12.1 verwendet werden, die Licht mit einem Intensitätsmaximum im UVA-Bereich von 365 nm erzeugt. Die Einstrahlung erfolgt mit einer Intensität, die keine akute Schädigung in der Haut verursacht, was unterhalb der einfachen MED, bzw. unterhalb der MZB-Werte, bzw. deutlich unterhalb den durch Sonneneinstrahlung verursachten Werten liegt. Das von der Haut des Probanden 3 remittierte Licht wird durch den Lichtleiter 4.2 an die Photodiode 13.1 der Detektoreinheit 13 geleitet, von der Photodiode 13.1 erfasst und in Messwerte umgewandelt, die Messwerte werden an die erste Steuereinheit 2.1 gesendet und in der ersten Steuereinheit 2.1 gespeichert.

Die erste Steuereinheit 2.1 fragt dann ab 120, ob die zweite Messung der mit Schutzmittel behandelten Haut des Probanden 3 bereits durchgeführt wurde. Ist dies nicht der Fall, zeigt dies die erste Steuereinheit 2.1 an. Zur Durchführung der zweiten Messung 110 mit appliziertem Schutzmittel wird das Schutzmittel auf die Haut des Probanden 3 aufgetragen 130, z.B. nach ISO 24442 oder 24444 in der Menge von 2.0mg/cm$^2$ auf der zu prüfenden Hautoberfläche. Die Applikation 130 des Schutzmittels und die anschließende zweite Messung 110 wird an vergleichbarer Stelle der Messprobe 3, insbesondere auf derselben Stelle der Haut eines Probanden 3, durchgeführt, um die Reproduzierbarkeit der ersten und der zweiten Messung 110 zu gewährleisten. Ebenfalls zur Gewährleistung der Reproduzierbarkeit steuert die erste Steuereinheit 2.1 die LED 12.1 derart an, dass das von der LED 12.1 erzeugte Licht durch den Lichtleiter 4.1 auf die Haut des Probanden 3 geleitet wird, wobei Intensität und/oder Belichtungszeit von erster und zweiter Messung 110 aufeinander abgestimmt sind.

Falls die Abfrage 120 ergibt, dass zweite Messung 110 bereits erfolgt ist, erfolgt eine Evaluierung 140 des Schutzmittels. Dazu führt die Auswerteeinheit 10 ein Programm zur Berechnung des Reflexionsspektrums $T_{in\ vivo}$ nach Gleichung 1 aus:

$$T_{in\,vivo} = \frac{1}{SPF_{in\,vivo}} = \sqrt{\frac{R}{R_0}}$$

Gl. 1

mit $T_{in\,vivo}$ als Funktion der Wellenlänge $\lambda$, $SPF_{in\,vivo}$ der Schutzfaktor ermittelt durch das in vivo Verfahren 100, $R_0$ reflektierte Intensität der unbehandelten Haut des Probanden 3 als Funktion der Wellenlänge $\lambda$, R reflektierte Intensität der mit Schutzmittel behandelten Haut des Probanden 3 als Funktion der Wellenlänge $\lambda$. Das hier dargelegte Verfahren 100 benötigt einen Zeitaufwand von einigen wenigen s bis einige 10s.

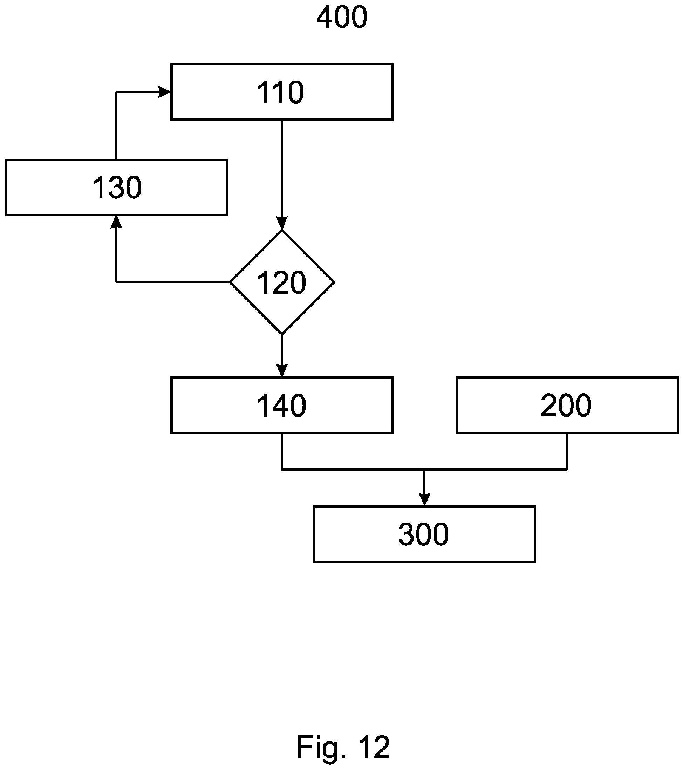

Fig. 12 zeigt ein Ausführungsbeispiel des erfindungsgemäßen Verfahrens 400 zur Bestimmung eines Schutzfaktors eines Hautschutzmittels. Dabei wird eine in vivo Reflexionsmessung 100 (s. Fig. 11) kombiniert mit den Daten eines Transmissionsspektrums 200. Erfindungsgemäß sind die Werte des Transmissionsspektrums 200 berechnet in silico und/oder in vitro gemessen.

Wenn die Mengen und Eigenschaften der UV-Filtersubstanzen eines Schutzmittels bekannt sind, kann die UV-Transmission berechnet werden, wobei auch die Unregelmäßigkeit des Films und dessen Photodegradation berücksichtigt sind. Anhand der simulierten UV-Transmission können die Schutzfähigkeit des Schutzmittels und alle Größen, die den Schutz gegen UVA- und/oder UVB-Strahlung charakterisieren, in silico berechnet werden.

Die in silico Bestimmung der Daten eines Transmissionsspektrums wird z.B. in einem Labor in Anlehnung an ISO 24443 durchgeführt. Angenommen wird eine Auftragung von 2,0g/cm² Schutzmittel. Die abgegebene Strahlung liegt in diesem Ausführungsbeispiel im Wellenlängenbereich von 280nm bis 500nm (UVB bis blaues Licht). In weiteren Ausführungen kann der Bestrahlungswellenlängenbereich im Wellenlängenbereich zwischen 280nm und 2000nm und bevorzugt zwischen 280nm bis 800nm liegen.

Der evaluierte Wellenlängenbereich des Transmissionsspektrums umfasst den Wellenlängenbereich von 280nm bis 2000nm, bevorzugt den Wellenlängenbereich von 280nm bis 800nm oder besonders bevorzugt den Wellenlängenbereich von 280nm bis 500nm und/oder den Wellenlängenbereich von 400nm bis 500nm und/oder den Wellenlängenbereich von 400nm bis 450nm. In diesem Ausführungsbeispiel umfasst der Evaluationswellenlängenbereich des Transmissionsspektrums 280nm bis 500nm.

Die Daten des in silico- und/oder in vitro Transmissionsspektrums werden in einer Datenbank gespeichert und sind von der Datenbank jederzeit abrufbar. Zur Ermittlung des Schutzfaktors eines Hautschutzmittels wird das in silico- und/oder in vitro Transmissionsspektrum in die Auswerteeinheit 10 geladen 200, die mit der Datenbank über eine Internet-Verbindung verbunden ist.

Die Ergebnisse der Evaluierungen des in vivo-Remissions-Spektrums 140 und des in silico- und/oder in vitro Transmissionsspektrums werden mittels der Auswerteeinheit 10 kombiniert evaluiert 300. Dazu wird mittels eines geeigneten Computer-Programms auf der Auswerteeinheit 10 zuerst das hybride Transmissionsspektrum $T_{hyb}$, berechnet, wobei das in silico-Transmissionsspektrum $T_{in\,silico}$ und/oder in vitro Transmissionsspektrum $T_{in\,vitro}$ mittels des Reflexionsspektrums $T_{in\,vivo}$ skaliert wird:

$$T_{hyb}(\lambda) = T_{in\,silico\,/in\,vitro}(\lambda) * \frac{T_{in\,vivo}(\lambda_{LED})}{T_{in\,silico\,/in\,vitro}(\lambda_{LED})} \text{Gl. 3}$$

Die Schutzfähigkeit des Schutzmittels für den Spektralbereich von UVA (320nm) bis in den HEV Spektralbereich (450nm) SF ergibt sich dann nach Gleichung 4 (hier ist E = IPD($\lambda$) das IPD Spektrum; S = I($\lambda$) ist das Sonnenspektrum):

$$SF = \frac{\int_{320}^{450} S(\lambda)E(\lambda)d\lambda}{\int_{320}^{450} S(\lambda)E(\lambda)T_{hyb}\,d\lambda}$$

Gl. 4

Die Schutzfähigkeit des Schutzmittels in dem Spektralbereich des blauen Lichts (400nm bis 500nm) wird ermittelt nach Gleichung 5 (hier ist E = IPD($\lambda$) das IPD Spektrum; S = I($\lambda$) ist das Sonnenspektrum)

$$SF(400nm - 500nm) = \frac{\int_{400}^{500} S(\lambda)E(\lambda)d\lambda}{\int_{400}^{500} S(\lambda)E(\lambda)T_{hyb}\,d\lambda}$$

Gl.5

Die Schutzfähigkeit des Schutzmittels von 400nm bis 450nm SF (400nm-450nm) wird nach Gleichung 6 ermittelt (hier ist E = IPD($\lambda$) das IPD Spektrum; S = I($\lambda$) ist das Sonnenspektrum):

$$SF(400nm - 450nm) = \frac{\int_{400}^{450} S(\lambda)E(\lambda)d\lambda}{\int_{400}^{450} S(\lambda)E(\lambda)T_{hyb}\,d\lambda}$$

Gl.6

Die Schutzfähigkeit des Schutzmittels für den Spektralbereich von UVA (320-400nm) UVA-SF ergibt sich dann nach Gleichung 4 (hier ist E = PPD($\lambda$) das PPD Spektrum; S = I($\lambda$) ist das Sonnenspektrum bzw. UVA-Quelle für PPD-test.):

$$UVA - SF = \frac{\int_{320}^{400} S(\lambda)E(\lambda)d\lambda}{\int_{320}^{400} S(\lambda)E(\lambda)T_{hyb}\,d\lambda}$$

Gl. 7

Die Schutzfähigkeit des Schutzmittels für den Spektralbereich von UVB bis UVA (280-400nm) UV-SF ergibt sich dann nach Gleichung 4 (hier ist E ($\lambda$) das Erythemwirkungsspektrum; S ($\lambda$) ist das Spektrum des Sonnensimulators nach ISO 24444)

$$UV - SF = \frac{\int_{290}^{400} S(\lambda)E(\lambda)d\lambda}{\int_{290}^{400} S(\lambda)E(\lambda)T_{hyb}\,d\lambda}$$

Gl. 8

In allen Gleichungen für SF oder UVA-SR wird optional ein Korrekturfunktion F (SF) = SF_korr bzw. F (UVA-SF) = UVA-PF_korr verwendet, welcher z. B. hauttypabhängige Unterschiede beschreibt. Eine Korrekturfunktion macht Werte verschiedener Hauttypen vergleichbar und gibt einen korrigierten Wert $SF_{korr}$ oder $UVA\text{-}PF_{korr}$ aus.

F kann beispielsweise ein linearer Faktor (d.h. F(SF)=SF*C) oder eine exponentielle Funktion sein (d.h. F(SF)=SF$^C$). C kann hierbei z. B. vom Hauttyp oder dem ITA°-Wert abhängig sein. Wie an Gl. 4 gezeigt, können die Formeln in Gleichung 2 bis 8 folgendermaßen geschrieben werden:

$$SF_{korr} = F(SF) = F\left(\frac{\int_{320}^{450} S(\lambda)E(\lambda)d\lambda}{\int_{320}^{450} S(\lambda)E(\lambda)T_{hyb}\,d\lambda}\right)$$

Gl. 9

Da der in vivo Wert ohne Photodegradation gemessen wird, sollte die Photodegradation geeignet berücksichtigt werden. Dies kann so erfolgen, dass z. B. $T_{\_in\ silico}$ mit ($T_{in\ silico\_irr}(\lambda)$ und ohne Photodegradation berechnet wird und ein spektraler Quotient daraus berechnet wird (analoges gilt für $T_{\_in\ vitro}$)

$$SRPD(\lambda) = \frac{T_{in\,silico\_irr}(\lambda)}{T_{in\,silico}(\lambda)}.$$

Gl. 10

Damit wird dann

$$T_{hyb\_irr}(\lambda) = T_{hyb}(\lambda) * SRPD(\lambda)$$

Gl.11

berechnet.

Die Kalibration der Methode kann beispielsweise durch geeignete Referenzverfahren wie die Elektronenspinresonanzspektroskopie erfolgen.

BEZUGSZEICHENLISTE

| | |
|---|---|
| 1 | Schutzfaktor-Evaluierungssystem |
| 2.1 | Erste Steuereinheit |
| 2.2 | Zweite Steuereinheit |
| 3 | Messkörper |
| 4.1, 4.2 | Lichtleiter/Faserbündel |
| 5 | Probenkopf |
| 5.1 | Auslass |
| 5.2 | Einlass |
| 6 | Einhausung des Probenkopfes |
| 7 | Verbindung Schutzfaktor-Evaluierungshandgerät - Zweite Baueinheit |
| 10 | Auswerteeinheit |
| 11 | Ausgabeeinrichtung |
| 12 | Strahlquelleneinrichtung |
| 12.1 | Strahlquelle / LED |
| 13 | Detektoreinheit |
| 13.1 | Detektor / Photodiode |
| 14 | Bedieneinheit |
| 30 | Erste Baueinheit / Schutzfaktor-Evaluierungshandgerät |
| 31 | Erste Einhausung |
| 40 | Zweite Baueinheit / Mobilgerät |
| 41 | Zweite Einhausung |
| 100 | Durchführung einer Reflexionsmessung |
| 110 | Durchführung einer Kalibriermessung |
| 120 | Abfrage |
| 130 | Auftragen eines Schutzmittels |
| 140 | Auswertung |
| 200 | Einlesen Transmissionsspektrum |
| 300 | Bestimmung des Schutzfaktors |
| 400 | Verfahren zur Bestimmung eines Schutzfaktors eines Hautschutzmittels |
| COM1 | Erste Kommunikationseinheit |
| COM2 | Zweite Kommunikationseinheit |

**Patentansprüche**

**1.** Schutzfaktor-Evaluierungssystem (1) zur Bestimmung eines Schutzfaktors eines Hautschutzmittels mit

• einer Strahlungsquelle (12) mit

   - genau einer LED (12.1),

• einer Detektoreinheit (13) mit

   - genau einer Photodiode (13.1) und

• einer Steuereinheit (2.1),
• einer Auswerteeinheit (10).

**2.** Schutzfaktor-Evaluierungssystem (1) zur Bestimmung eines Schutzfaktors eines Hautschutzmittels nach Anspruch 1,
**dadurch gekennzeichnet, dass**
die Strahlungsquelle (12) und die Detektoreinheit (13) in einer ersten Baueinheit (30) zusammengefasst sind.

**3.** Schutzfaktor-Evaluierungssystem (1) zur Bestimmung eines Schutzfaktors eines Hautschutzmittels nach Anspruch 1 oder 2,
**dadurch gekennzeichnet, dass**
die erste Baueinheit (30) eine erste Kommunikationseinheit (COM1) aufweist.

**4.** Schutzfaktor-Evaluierungssystem (1) zur Bestimmung eines Schutzfaktors eines Hautschutzmittels nach einem oder mehreren der vorhergehenden Ansprüche,
**dadurch gekennzeichnet, dass**
die erste Baueinheit (30) und die Steuereinheit (2.1) oder die Auswerteeinheit (10) in unterschiedlichen Einhausungen (31, 41) angeordnet sind.

**5.** Schutzfaktor-Evaluierungssystem (1) zur Bestimmung eines Schutzfaktors eines Hautschutzmittels nach Anspruch 4,
**dadurch gekennzeichnet, dass**
die unterschiedlichen Einhausungen (31, 41) keine strukturelle Verbindung miteinander aufweisen.

**6.** Schutzfaktor-Evaluierungssystem (1) zur Bestimmung eines Schutzfaktors eines Hautschutzmittels nach einem oder mehreren der vorhergehenden Ansprüche,
**dadurch gekennzeichnet, dass**
die erste Baueinheit (30) die Steuereinheit (2.1) aufweist.

**7.** Schutzfaktor-Evaluierungssystem (1) zur Bestimmung eines Schutzfaktors eines Hautschutzmittels nach einem oder mehreren der vorhergehenden Ansprüche,
**dadurch gekennzeichnet, dass**
die Auswerteeinheit (10) in einer zweiten Baueinheit (40) angeordnet ist.

**8.** Schutzfaktor-Evaluierungssystem (1) zur Bestimmung eines Schutzfaktors eines Hautschutzmittels nach Anspruch 7,
**dadurch gekennzeichnet, dass**
die zweite Baueinheit (40) eine zweite Kommunikationseinheit (COM2) aufweist.

**9.** Schutzfaktor-Evaluierungssystem (1) zur Bestimmung eines Schutzfaktors eines Hautschutzmittels nach Anspruch 8,
**dadurch gekennzeichnet, dass**
die erste Kommunikationseinheit (COM1) und die zweite Kommunikationseinheit (COM2) geeignet sind, miteinander zu kommunizieren.

**10.** Schutzfaktor-Evaluierungssystem (1) zur Bestimmung eines Schutzfaktors eines Hautschutzmittels nach einem oder mehreren der vorhergehenden Ansprüche,
**dadurch gekennzeichnet, dass**
die LED (12.1) geeignet ist, Licht mit einer Wellenlänge im UVA-Bereich zu emittieren.

**11.** Schutzfaktor-Evaluierungssystem (1) zur Bestimmung eines Schutzfaktors eines Hautschutzmittels nach einem oder mehreren der vorhergehenden Ansprüche,
**dadurch gekennzeichnet, dass**
die erste Baueinheit (30) ein Handgerät ist.

**12.** Schutzfaktor-Evaluierungssystem (1) zur Bestimmung eines Schutzfaktors eines Hautschutzmittels nach einem oder mehreren der vorhergehenden Ansprüche,
**dadurch gekennzeichnet, dass**
die zweite Baueinheit (40) ein Mobilgerät und bevorzugt ein Smartphone ist.

**13.** Verfahren (400) zur Bestimmung eines Schutzfaktors eines Hautschutzmittels mit den Verfahrensschritten:

- Emittieren von Strahlung aus genau einer LED (12.1) einer Strahlungsquelle (12),
- Detektieren von remittierter Strahlung von genau einer Photodiode (13.1) einer Detektoreinheit (13),
- Evaluierung (300) des Schutzfaktors in einem Evaluierungswellenlängenbereich,
wobei der Schutzfaktor des Schutzmittels aus der remittierten Strahlung und eines Transmissionsspektrums evaluiert (300) wird,
wobei die Daten des Transmissionsspektrums zur Bestimmung des Schutzfaktors in-silico- und/oder in-vitro-Daten sind.

**14.** Verfahren (400) zur Bestimmung eines Schutzfaktors eines Hautschutzmittels nach Anspruch 13,
**dadurch gekennzeichnet, dass**
Daten der detektierten remittierten Strahlung zu einer Auswerteeinheit (10) übertragen werden.

**15.** Verfahren (400) zur Bestimmung eines Schutzfaktors eines Hautschutzmittels nach Anspruch 14,
**dadurch gekennzeichnet, dass**
die Auswerteeinheit (10) in ein anderen strukturellen Baueinheit (30, 40) angeordnet ist als die Strahlungsquelle (12.1) und/oder die Detektoreinheit (13).

**16.** Verfahren (400) zur Bestimmung eines Schutzfaktors eines Hautschutzmittels nach einem oder mehreren der Ansprüche 13 bis 15,
**dadurch gekennzeichnet, dass**
die emittierte Strahlung einen Bestrahlungswellenlängenbereich zwischen 280 nm und 2000 nm, bevorzugt 280 nm bis 800 nm und besonders bevorzugt den Bereich von 280 nm bis 500 nm umfasst,

**17.** Verfahren (400) zur Bestimmung eines Schutzfaktors eines Hautschutzmittels nach Anspruch 16,
**dadurch gekennzeichnet, dass**
der Evaluierungswellenlängenbereich zum Bestrahlungswellenlängenbereich unterschiedlich ist.

**18.** Verfahren (400) zur Bestimmung eines Schutzfaktors eines Hautschutzmittels nach einem oder mehreren der Ansprüche 13 bis 17,
**dadurch gekennzeichnet, dass**
der Schutzfaktor des Schutzmittels aus der remittierten Strahlung und eines Transmissionsspektrums evaluiert (300) wird.

**19.** Verfahren (400) zur Bestimmung eines Schutzfaktors eines Hautschutzmittels nach einem oder mehreren der Ansprüche 13 bis 18,
**dadurch gekennzeichnet, dass**
die Auswerteeinheit (10) ein Computer oder ein Mobilfunkgerät ist.

**20.** Verfahren (400) zur Bestimmung eines Schutzfaktors eines Hautschutzmittels nach einem oder mehreren der Ansprüche 13 bis 19,
**dadurch gekennzeichnet, dass**
die Strahlquelle (12.1) und/oder die Detektoreinheit (13) in einem Handgerät (30) angeordnet sind.

1

Fig. 1

Fig. 2

1

Fig. 3

1

Fig. 4

Fig. 5

Fig. 6

Fig. 7

Fig. 8

Fig. 9

EP 4 372 364 A1

1

Fig. 10

23

100

Fig. 11

400

Fig. 12

Europäisches
Patentamt

European
Patent Office

Office européen
des brevets

# EUROPÄISCHER RECHERCHENBERICHT

**Nummer der Anmeldung**

**EP 23 21 0407**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | KR 101 749 703 B1 (PARK SANG MYUNG [KR]; PARK SANG GEOL [KR]) 21. Juni 2017 (2017-06-21) | 1-12 | INV. G01N21/31 G01N21/84 |
| Y | * Absätze [0033], [0040] - [0071]; Abbildungen 1,2 * | 1-20 | ADD. G01N21/01 |
| Y | OSTERWALDER ULI ET AL: "Charakterisierung von Sonnenschutzleistung: Quo vadis?", HAUTARZT, SPRINGER VERLAG, BERLIN, DE, Bd. 73, Nr. 4, 25. März 2022 (2022-03-25), Seiten 276-282, XP037774510, ISSN: 0017-8470, DOI: 10.1007/S00105-022-04958-X [gefunden am 2022-03-25] * Seite 279, mittlere Spalte - rechte Spalte; Abbildung 3 * * Seite 277, mittlere Spalte * | 13-20 | G01N21/33 G01N21/47 |
| Y | WO 2020/232047 A1 (BEIERSDORF AG [DE]; MUENSTERER HERIBERT [US]) 19. November 2020 (2020-11-19) * Absätze [0001], [0018] - [0019], [0030] - [0039]; Anspruch 1; Abbildungen 1,2A,2B,3,4 * | 1-20 | **RECHERCHIERTE SACHGEBIETE (IPC)** G01N |
| X | GEORG WIORA ET AL: "Development of a fast non-invasive in vivo measurement of UVA-PF and SPF with new Diffuse Reflectance Spectroscopy device", IFSCC, 30. September 2019 (2019-09-30), XP055708127, * Zusammenfassung; Abbildung 4 * * das ganze Dokument * | 1,13 | |

-/--

Der vorliegende Recherchenbericht wurde für alle Patentansprüche erstellt

| Recherchenort | Abschlußdatum der Recherche | Prüfer |
|---|---|---|
| München | 15. März 2024 | Duijs, Eric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

Europäisches
Patentamt

European
Patent Office

Office européen
des brevets

**EUROPÄISCHER RECHERCHENBERICHT**

Nummer der Anmeldung

**EP 23 21 0407**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2022/240838 A1 (KOHLI INDERMEET [US] ET AL) 4. August 2022 (2022-08-04) * Absätze [0002], [0005], [0036] – [0038]; Abbildungen 1,2 * ----- | 16 | |
| Y | MOSELEY H ET AL: "New sunscreens confer improved protection for photosensitive patients in the blue light region", BRITISH JOURNAL OF DERMATOLOGY, JOHN WILEY, HOBOKEN, USA, Bd. 145, Nr. 5, 7. Juli 2008 (2008-07-07), Seiten 789-794, XP071116958, ISSN: 0007-0963, DOI: 10.1046/J.1365-2133.2001.04429.X * Zusammenfassung; Abbildungen 2-4 * ----- | 16 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| München | 15. März 2024 | Duijs, Eric |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 21 0407

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-03-2024

| Im Recherchenbericht angeführtes Patentdokument | Datum der Veröffentlichung | Mitglied(er) der Patentfamilie | | Datum der Veröffentlichung |
|---|---|---|---|---|
| KR 101749703 B1 | 21-06-2017 | KEINE | | |
| WO 2020232047 A1 | 19-11-2020 | KEINE | | |
| US 2022240838 A1 | 04-08-2022 | CA | 3146389 A1 | 14-01-2021 |
| | | EP | 3997383 A1 | 18-05-2022 |
| | | US | 2022240838 A1 | 04-08-2022 |
| | | WO | 2021007420 A1 | 14-01-2021 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19828497 A1
- DE 102004020644 A1